Europäisches Patentamt

European Patent Office

Office européen des brevets

(19)

(11) **EP 0 754 451 A1**

(12) **EUROPEAN PATENT APPLICATION**
published in accordance with Art. 158(3) EPC

(43) Date of publication:
22.01.1997 Bulletin 1997/04

(21) Application number: 96901350.7

(22) Date of filing: 26.01.1996

(51) Int. Cl.$^6$: **A61K 9/127**

(86) International application number:
PCT/ES96/00014

(87) International publication number:
WO 96/22763 (01.08.1996 Gazette 1996/35)

(84) Designated Contracting States:
CH DE FR GB IT LI NL

(30) Priority: 27.01.1995 ES 9500016
12.09.1995 ES 9500177

(71) Applicant: **Palomino Munoz, Juan Manuel**
28903 Getafe (ES)

(72) Inventor: **PALOMINO MUNOZ, Teodoro**
28025 Madrid (ES)

(74) Representative: **Garcia Cabrerizo, Francisco**
OFICINA GARCIA CABRERIZO S.L.
Vitruvio 23
28006 Madrid (ES)

(54) **LIPOSOME FOR DESTROYING HIV AND CELLS INFECTED BY SAID VIRUS**

(57) Liposome for destroying HIV and cells infected by said virus, which has one or various lipid layers, while having in their interior a protease or a RNAse, and having on their external surface molecules of the CD4 receptor, or analogs or fractions thereof, adhered thereto.

FIG. 1

Printed by Rank Xerox (UK) Business Services
2.13.13/3.4

## Description

The present invention consists in the development of a medication formed by liposomes with a RNAse and liposomes containing a protease (by means of the evaporation technique in reverse phase or another alternative method), covering the liposomes with CD4 for its administration to the infected patient.

## PRESENT STATE OF THE ART

The HIV has affinity for the CD4 molecule (present in the membrane of a definite type of lymphocyte T), being this the entry way of the virus in the cellular elements carrying such molecule.

The glucoprotein 120 (GP120) of the viral envelope interacts with the molecule CD4 present in the cellular membrane of the future host cell; after this interaction the penetration of the virus in the cell is produced, and the subsequent release of the nucleocapsid in the cytoplasm thereof. The decapsidation of this one releases the viral RNA and the reverse transcriptase enzyme (RT).

The reverse transcriptase synthesizes the single stranded DNA and subsequent double stranded DNA taking as mould the viral RNA. The double stranded DNA is integrated in the cellular genome; taking advantage of the energy and substrates of the host cell, it synthesizes the proteins and the RNA of the future viral nucleocapsid "clons". The viral proteins are transferred to the cells surface (like, the GP120 expressed in the cell membrane itself) in which, by exocytosis mechanism, the viral envelope is formed. This envelope contains GP120 viral molecules, which ensure the capacity of infection of the resulting virions.

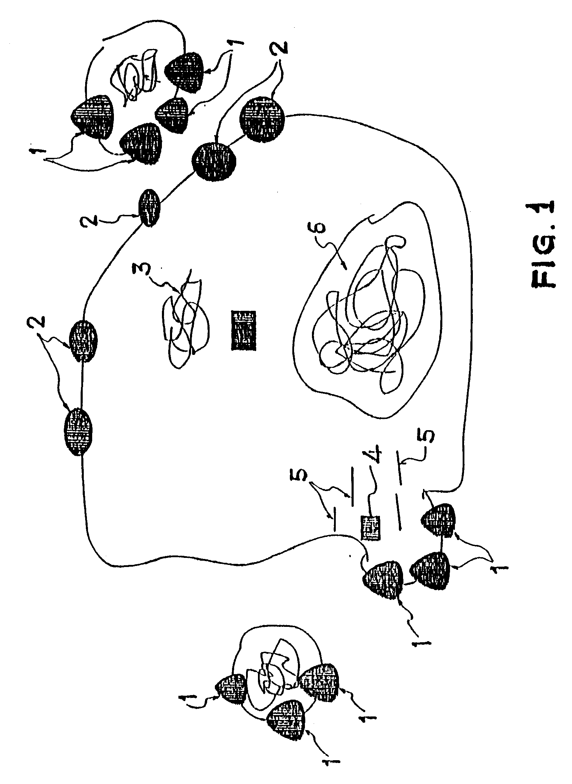

In fig. 1, we see in the right side a HI V with the CP120 (1), right at the moment of interacting with the CD4 (2) receptor; once it interacts, the virus penetrates the cell, releasing the viral RNA (3); such RNA passes through the RT (4) to double stranded DNA, integrating itself in the DNA of the host cell (6). The proteins of the nucleocapsid (5) GP120 (which are incorporated in the cellular membrane) and RT are synthesized. The virions emerge, to the left of the figure, through exocytosis with all the components to infect a new cell.

Besides all the mentioned above, the infected lymphocytes, having in their plasmatic membrane GP120, are capable of interacting with other healthy lymphocytes (link GP120-CD4 of the healthy lymphocyte) and forming lethal syncytia (that is, they drag, when destroying themselves many healthy lymphocytes linked to them).

This contributes to the high immunodeficiency of the infected patients.

The treatment against the virus is at present centered in the inhibition of the RT enzyme, via medication of the AZT type; against this medication the virus can become resistant. AZT has been combined with soluble CD4.

The viral tropism by the CD4 molecule has been the base of some therapeutic concept.

It is administered intravenously or subcutaneously, because it is a protein, with the purpose of competing with CD4 of the lymphocytes CD4 in the virus link, and in this way to avoid that the virus infects a new lymphocyte.

The HIV can link either with CD4 of the lymphocytes membrane as the soluble CD4 (administered as medication). Schematically we can present it as follows:

a)

$$HIV + CD4Lyn \rightarrow HIV\text{-}CD4Lyn$$

b)

$$HIV + CD4 \rightarrow HIV\text{-}CD4$$

where:

HIV = Virus
CD4 = soluble CD4
HIV-CD4 = Virus linked to soluble CD4
CD4Lyn = CD4 of the lymphocyte membrane
HIV-CD4Lyn = Virus linked to CD4 of the lymphocyte membrane.

a) and b) are related, they share the HIV component.

a) represents the non treated patient:
the HIV-CD4Lyn is lethal, and therefore disappears. CD4Lyn (Lymphocytes with CD4) will reach a phase so low that will endanger the life of the patient.

The group a) + b) represents the patient treated with soluble CD4.

The soluble CD4 links HIV in order to form HIV-CD4, competing with the healthy lymphocytes linked with the virus and in this way preventing the linking of the virus with the healthy lymphocytes; furthermore, the CD4 links to the infected lymphocytes (which have GP120 in their membrane) and prevents that these form lethal syncytia with other healthy lymphocytes. (The infected lymphocytes which make evident the GP120, within the nomenclature that we are using, would have to be considered as HIV; because they link and kill CD4 lymphocytes). According to the present invention the important aspect is, apart from blocking the viral GP120 and from the infected lymphocytes, to eliminate the product of the soluble link VIH-CD4 and the infected soluble lymphocyte-CD4 in an efficient way. That is, if we do not obtain an efficient elimination of the soluble HIV-CD4 complex and of the infected soluble lymphocyte-CD4, we are probably generating a new virus reservoir.

Such reason has taken us to invent a medication which simultaneously acts on HIV and infected lymphocyte.

The object of the present invention will be better understood by the following description:

The way of eliminating the virus consists in a liposomes medication with the absorbed CD4 molecule; liposomes which contain either a protease (Example Trypsin) or a RNAse (Example, Ribonuclease T).

When a liposome makes coalescence with a HIV particle the protease destroys its nucleocapsid; when making coalescence with the liposome that contains RNAse, this one destroys its RNA.

The infected cells (carriers of GP120) will be destroyed when making coalescence with liposomes, which are loaded with protease.

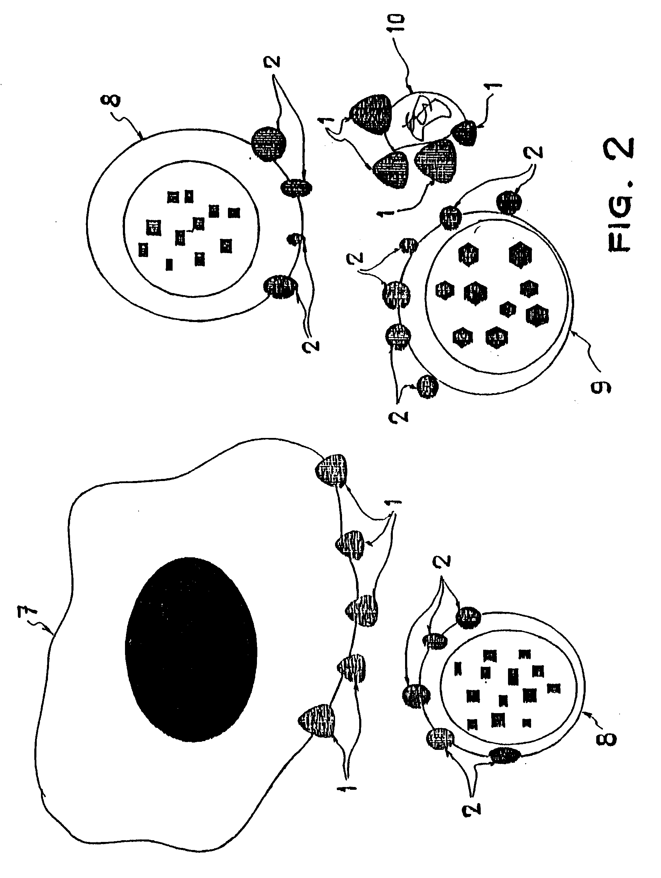

In figure 2 it is represented a schematic interaction of the liposome with virus and infected cell.

(7) Represents an infected cell, which expresses GP120 (1) (fig. 1) in its membrane.
(8) Represents a liposome with protease encapsulated molecules (rectangular shape), and in its external side molecules of adsorbed CD4 (2) (Fig. 1).
(9) Represents the liposome with encapsulated ribonuclease molecules (hexagonal shape), and in its external side molecules of adsorbed CD4 (2) (Fig. 1).
(10) Represents a HIV viral particle, with GP120 molecules (1) (Fig. 1) in its envelope.

MANUFACTURING PROCEDURE

1.- Lipid composition of the liposome:

- molar ratio: PC/CH/PE/PDP-PE 9.7: 5: 0.15: 0.2

where:

PC is egg phosphatidilcholine
CH is cholesterol
PE is trioleilphosphatidilethanolamine and
PDP-PE is N-[3-(2-piridilditio) propionil] dipalmitoil phosphatidiletanomine.

2.- Production of liposome and encapsulation of protease (trypsin) and RNAse (ribonuclease T) by means of evaporation in reverse phase, for example:

The liposomes prepared by means of evaporation in reverse phase, in buffer 20 mM Mes, 20 mM Mops, 125 mM of ClNa, 1 mM EDTA (pH 6.7), trypsin 8 mg/ml, or ribonuclease T 8 mg/ml and extrusion through the membranes of double polycarbonate (0.1 micrometer of porous diameter, varying the membrane size of the pore we can obtain liposomes of larger size) under pressure of argon in a high pressure steel extrusor.

3.- Elimination of trypsin, or ribonuclease T non encapsulated:

Both types of liposomes are purified in discontinuous flotation gradient of metrizamide, followed by the overnight dialysis (Spectrapor 2) against amounts as minimum of 1000 buffer volumes (20 mM Mes, 20 mM Mops, 125 mM NaCl (pH 6.7) under argon atmosphere, at 4°C.

4.- Coupling of the soluble recombining CD4 receptor: $rsCD_4$.

It can be carried out in many ways, as for example through the thiolation method (SATA), either with the MBPH method (4-(4-N-maleimidophenyl butyric hydrazide) acid, which we describe below.

5.4 mg/ml of $rsCD_4$ in 0.1 mM of sodium acetate, pH 5.5 oxided 30' with sodium periodate 10 mM at room temperature (finally eliminated with filtration in Sephadex G-25, balanced with sodium acetate 0.1 M, pH 5.5).

MPBH in dimethylformamide is added to oxided $rsCD_4$ during 2 hours at room temperature (final concentrations 1 mM and 2.7 mg/ml respectively); the MPBH is removed by filtration in Sephadex G-25 (balanced with sodium acetate 10 mM/sodium chloride 150 mM, pH 5.5). We concentrate the conjugated at 10 mg/ml in a protein concentrator (example Centricon 10 (Amicon).

The liposomes are reduced with 12 mM of DTT (dithiothreitol), at room temperature during 1 h, under argon atmosphere, and the excess of dithiotreitol is eliminated by filtration in Sephadex G-75 balanced with buffer (20 mM Mes, 20 mM Mops, 125 mM ClNa, 1 mM of EDTA (pH 6.7)), under constant argon jet.

Immediately before mixing the $rsCD_4MBPH$ with the liposomes, we adjust its pH at 7.0 (with 500 mM of Mes/500 mM of Mops, pH 8.0). The coupling is obtained for final concentrations of $rsCD_4$ 2.4 mg/ml and liposome 10 mM of lipid, under argon atmosphere, room temperature, with soft stirring during overnight.

For the final purification of the coupling liposomes we refer to point 3.

To the encapsulated trypsin fraction, we add 1 mg of Trypsin Inhibitor per each millilitre of liposomes solution, in this way avoiding that the leakage of the liposome protease damages the CD4 receptor of the liposome surface.

In this way we obtain the medication consisting in liposomes of an approximate size of 100 and 400 nanometres, covered in their surface with CD4 and containing a protease, or both liposomes with CD4 in their surface and a RNAse inside.

## Claims

1. Liposome for the destruction of HIV and of the infected cells thereof, characterized in that it has one or various lipid layers, provided that they contain a protease or a RNAse, and in their external surface have a CD4 receptor molecules or analogous thereof or fractions thereof adhered.

2. Liposome, according to the previous claim, characterized in that there are one or various lipid layers, which are formed by the usual lipids in the liposomes manufacturing, provided that they contain protease or a RNAse and in their external surface they have CD4 receptor molecules or analogous thereof or fractions thereof adhered.

3. Liposome, according to claims 1 and 2, characterized in that the protease is a trypsin.

4. Liposome, according to claims 1 and 2, characterized in that the RNAse is a ribonuclease T.

FIG. 1

FIG. 2

## INTERNATIONAL SEARCH REPORT

International application No.

PCT/ES 96/00014

### A. CLASSIFICATION OF SUBJECT MATTER

Int. Cl.$^6$    A61K9/127

According to International Patent Classification (IPC) or to both national classification and IPC

### B. FIELDS SEARCHED

Minimum documentation searched (classification system followed by classification symbols)

Int. Cl.$^6$    A61K

Documentation searched other than minimum documentation to the extent that such documents are included in the fields searched

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | DE,A 37 11 724 (HARRO BOERNER MEDIZINISCHE KOMMUNIKATION GMBH) 20 October 1988<br>see claims 1-3; column 2, line 65; column 3 line 25 | 1,2 |
| Y | WO,A, 90 00555 (VICAL INC.) 25 January 1990<br>see page 6, line 30; page 7, line 4; page 8, lines 25-32 | 1,2 |

☐ Further documents are listed in the continuation of Box C.    ☒ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 24 April 1996 (24.04.96) | 14 May 1996 (14.05.96) |

| Name and mailing address of the ISA/<br>O.E.P.M. | Authorized officer |
|---|---|
| Facsimile No. | Telephone No. |

Form PCT/ISA/210 (second sheet) (July 1992)